# EUROPEAN PATENT APPLICATION

(11) **EP 4 796 135 A1**
(43) Date of publication of application: **26.08.2026**
(21) Application number: 23958730.6
(22) Date of filing: 08.12.2023
(51) Int. Cl.: A61L 24/06

(54) **POLYVINYL ALCOHOL EMBOLIC MICROSPHERE WITH CORE-SHELL STRUCTURE, PREPARATION METHOD THEREFOR, AND USE THEREOF**

(30) Priority: 17.11.2023 CN 202311551133
(71) Applicant: Cardiolink Science (Shenzhen) Medical Technology Development Co., Ltd., Shenzhen, Guangdong 518118 (CN)
(72) Inventor: SUN, Hongtao, Shenzhen, Guangdong 518118 (CN); YAO, Lijuan, Shenzhen, Guangdong 518118 (CN); LIU, Meifang, Shenzhen, Guangdong 518118 (CN); YANG, Liuhong, Shenzhen, Guangdong 518118 (CN); ZHANG, Changzhong, Shenzhen, Guangdong 518118 (CN); SUN, Peng, Shenzhen, Guangdong 518118 (CN)
(74) Representative: Tiburzi, Andrea
(86) International application number: PCT/CN2023/137426
(87) International publication number: WO 2025/102461

(57) **Abstract**

Disclosed are a polyvinyl alcohol embolic microsphere with a core-shell structure, a preparation method therefor, and use thereof. According to the preparation method, polyvinyl alcohol is taken as a skeleton material, two modified polyvinyl alcohols with different grafting degrees are obtained by means of reacting N-(2,2-dimethoxy)-2-methylacrylamide with polyvinyl alcohol at different ratios, a water-soluble monomer with ionic functional groups and an initiator are mixed with an aqueous solution of low-grafting-degree polyvinyl alcohol to obtain a first aqueous phase, an initiator is mixed with an aqueous solution of high-grafting-degree polyvinyl alcohol to obtain a second aqueous phase, the two aqueous phases are added to an oil phase containing an oil-soluble dispersing agent to form a water-in-oil reverse-phase suspension polymerization system, and reverse-phase suspension polymerization is performed to obtain the polyvinyl alcohol embolic microsphere with a core-shell structure. The core-shell structure polyvinyl alcohol embolization microsphere exhibits an extremely fast drug loading rate and an extremely high drug loading.

## Description

### TECHNICAL FIELD

The present application belongs to the technical field of embolic microspheres, specifically relates to a core-shell structured polyvinyl alcohol embolic microsphere, a preparation method therefor, and use thereof.

### BACKGROUND ART

Currently, for marketed embolic microspheres, polyvinyl alcohol is mainly used as a skeleton material of the microspheres. By grafting monomers with double bonds onto a molecular chain of polyvinyl alcohol, and then polymerizing and cross-linking the monomers with monomers with ionic functional groups, charged embolic microspheres are formed, thereby achieving a function of drug loading. The microspheres prepared by the single cross-linked structure possess certain mechanical properties (strength and compressive elasticity) and drug-loading function. Repulsive forces between internal charges in the microspheres provide some support for structures of the microspheres. However, when the microspheres are loaded with oppositely charged drugs through electrostatic interactions, the internal repulsive forces in the microspheres disappear due to charge neutralization. This leads to a sharp volume shrinkage of the microspheres, which in turn affects further diffusion of the drugs into interior of the microspheres and ultimately results in a relatively low drug loading.

CN201410232150 discloses a preparation method for single-crosslinked polyvinyl alcohol embolic microsphere. According to the method, polyvinyl alcohol is used as a skeleton material, and a cross-linking agent, acrylamido-dimethoxyacetal, was first grafted onto polyvinyl alcohol to obtain a modified polyvinyl alcohol. Then, sodium 2-acrylamido-2-methylpropanesulfonate monomer, an initiator (potassium persulfate), and the modified polyvinyl alcohol were mixed to obtain an aqueous phase. Finally, using tetramethylethylenediamine as a catalyst, butyl acetate as a solvent, and cellulose acetate butyrate as a dispersant, reverse-phase suspension polymerization was performed to obtain the microspheres. Compressive elasticity of the microspheres can reach over 50%, but a time required for doxorubicin loading to reach equilibrium is 20 min, which means that doctors need to wait at least 20 min before clinical use. Furthermore, a drug loading is only about 50%, which may lead to poor therapeutic efficacy.

CN201910504935 discloses a preparation method for gradient-crosslinked polyvinyl alcohol embolic microspheres. According to the method, polyvinyl alcohol is used as a skeleton material, directly prepared into an aqueous solution. Then sodium 2-acrylamido-2-methylpropanesulfonate monomer, a crosslinking agent (N,N-methylenebisacrylamide), and an initiator (potassium persulfate) were added to form an aqueous phase. After that, tetramethylethylenediamine is used as a catalyst, butyl acetate is used as a solvent, cellulose acetate butyrate is used as a dispersant, and glutaraldehyde was added in batches as a crosslinking agent during reverse-phase suspension polymerization process to obtain gradient-crosslinked microspheres. Compressive elasticity of the microspheres can reach over 70%, but a drug loading can reach 98%. However, a duration of reaching the drug loading requires 30 min, which also means that doctors need to wait at least 30 min before clinical use.

Therefore, there is a need in the field to further develop an embolic microsphere with a shorter drug loading equilibrium time and a higher drug loading.

### SUMMARY

The present application provides a core-shell structured polyvinyl alcohol embolic microsphere, a preparation method therefor, and use thereof.

In the first aspect, the present application provides a preparation method for a core-shell structured polyvinyl alcohol embolic microsphere, and the preparation method includes following steps:
(1) reacting polyvinyl alcohol with a water-soluble crosslinking agent accounting for 2%-8% (such as, 2%, 3%, 5%, 7% or 8%) by mass of the polyvinyl alcohol to obtain an aqueous solution of low-grafting-degree polyvinyl alcohol;
(2) reacting polyvinyl alcohol with a water-soluble crosslinking agent accounting for 16%-32% (such as, 16%, 18%, 20%, 25%, 28%, 30% or 32%) by mass of the polyvinyl alcohol to obtain an aqueous solution of high-grafting-degree polyvinyl alcohol;
(3) mixing a water-soluble monomer with ionic functional groups, an initiator and the aqueous solution of low-grafting-degree polyvinyl alcohol obtained in step (1) to obtain a first aqueous phase;
(4) mixing an initiator with the aqueous solution of high-grafting-degree polyvinyl alcohol obtained in step (2) to obtain a second aqueous phase;
(5) mixing the first aqueous phase and the second aqueous phases to obtain a mixed aqueous phase; and
(6) adding the mixed aqueous phase obtained in step (5) to an oil phase containing an oil-soluble dispersant to form a water-in-oil system for reverse-phase suspension polymerization, and performing reverse-phase suspension polymerization to obtain the core-shell structured polyvinyl alcohol embolic microsphere.

In the present application, an aqueous solution of low-grafting-degree polyvinyl alcohol is obtained in step (1), and an aqueous solution of high-grafting-degree polyvinyl alcohol is obtained in step (2). A microsphere with a core-shell structure is obtained by reversed-phase suspension polymerization of water phases of two modified polyvinyl alcohols with different grafting degrees with an oil phase.

The low-grafting-degree modified polyvinyl alcohol in the first aqueous phase has a higher degree of hydrophilicity. During a process of the reverse suspension polymerization to form droplet, the low-grafting-degree modified polyvinyl alcohol and the water-soluble monomer with ionic functional groups are distributed inside the droplet to form a core. While the high-grafting-degree modified polyvinyl alcohol in the second aqueous phase has a relatively lower degree of hydrophilicity. During the process of the reverse suspension polymerization to form droplets, the high-grafting-degree modified polyvinyl alcohol tends to distribute on the surface of the droplet to form a shell, thus forming a droplet with a core-shell structure. The droplet is then subjected to cure through a reaction to form a microsphere with a core-shell structure.

A relatively high cross-linking degree of the shell reduces a particle size shrinkage degree of the microsphere after drug loading to a certain extent. The shell can still maintain relatively unobstructed channels, facilitating rapid entry of drugs from the shell into the core and improving drug loading rate.

The shell contains almost no water-soluble monomer with ionic functional groups, while the core is rich in the water-soluble monomer with ionic functional groups, forming a charge difference between the inside and outside. Under the positive attraction of the core, negatively charged drugs are more easily to enter the core, thus improving utilization rate of sodium 2-acrylamido-2-methylpropanesulfonate, thereby further increasing drug loading.

The unique core-shell structure enables the microsphere to have an extremely fast drug loading rate and an extremely high drug loading. Compared to conventional drug loading (80 mg, 2 mL microspheres), the microsphere with such a structure can achieve a drug loading of over 95% in 1 min, with a maximum drug loading capacity of 150 mg.

In some embodiments, before the reaction in step (1), polyvinyl alcohol is added to water, heated to 90 °C-100 °C (such as, 90 °C, 93 °C, 95 °C, 98 °C or 100 °C), and stirred until dissolved to form a homogeneous polyvinyl alcohol solution.

In some embodiments, a mass percentage concentration of polyvinyl alcohol in the polyvinyl alcohol solution is 12%-22%, such as, 12%, 15%, 18%, 20% or 22%.

In some embodiments, the water-soluble crosslinking agents in step (1) and step (2) are each independently at least one selected from the group consisting of N-(2,2-dimethoxy)-2-methacrylamide, N-(1-hydroxy-2,2-dimethoxyethyl)-2-acrylamide, N-(2,2-dimethoxy)-2-acrylamide, N-(1-hydroxy-2,2-dimethoxyethyl)-2-methacrylamide, N-(2,2-dimethoxy)-2-methacrylate, N-(1-hydroxy-2,2-dimethoxyethyl)-2-acrylate, N-(2,2-dimethoxy)-2-acrylate, and N-(1-hydroxy-2,2-dimethoxyethyl)-2-methacrylate.

In some embodiments, the reaction in step (1) is performed in the presence of an acid catalyst.

In some embodiments, the acid catalyst is at least one selected from the group consisting of hydrochloric acid, sulfuric acid, nitric acid, and p-toluenesulfonic acid, preferably hydrochloric acid.

In some embodiments, a mass percentage concentration of the acid catalyst in the reaction system is 6%-12%, such as, 6%, 7%, 8%, 9%, 10%, 11% or 12%.

In some embodiments, a temperature of the reaction in step (1) is 20 °C-30 °C (such as, 20 °C, 23 °C, 25 °C, 28 °C or 30 °C), and a duration of the reaction is 10 h-15 h (such as, 10 h, 11 h, 12 h, 13 h, 14 h or 15 h).

In some embodiments, before the reaction in step (2), polyvinyl alcohol is added to water, heated to 90 °C-100 °C (such as, 90 °C, 93 °C, 95 °C, 98 °C or 100 °C), and stirred until dissolved to form a homogeneous polyvinyl alcohol solution.

In some embodiments, a mass percentage concentration of polyvinyl alcohol in the polyvinyl alcohol solution is 12%-22%, such as,12%, 15%, 18%, 20% or 22%.

In some embodiments, the reaction in step (2) is performed in the presence of an acid catalyst.

In some embodiments, the acid catalyst is at least one selected from the group consisting of hydrochloric acid, sulfuric acid, nitric acid, and p-toluenesulfonic acid, preferably hydrochloric acid.

In some embodiments, a temperature of the reaction in step (2) is 20 °C-30 °C (such as, 20 °C, 23 °C, 25 °C, 28 °C or 30 °C), and a duration of the reaction is 10 h-15 h (such as, 10 h, 11 h, 12 h, 13 h, 14 h or 15 h).

In some embodiments, the water-soluble monomer with ionic functional groups in step (3) is at least one selected from the group consisting of sodium 2-acrylamido-2-methylpropanesulfonate, sodium allyl sulfonate, and sodium methallyl sulfonate.

In some embodiments, an amount of the water-soluble monomer with ionic functional groups in step (3) is 5%-20%, such as, 5%, 8%, 10%, 12%, 15%, 18% or 20%, by weight of the aqueous solution of polyvinyl alcohol.

In some embodiments, the initiator in step (3) is at least one selected from the group consisting of ammonium persulfate, sodium persulfate, and potassium persulfate.

In some embodiments, an amount of the initiator in step (3) is 0.3%-0.8%, such as, 0.3%, 0.4%, 0.5%, 0.6%, 0.7% or 0.8%, by weight of the aqueous solution of polyvinyl alcohol.

In some embodiments, the initiator in step (4) is at least one selected from the group consisting of ammonium persulfate, sodium persulfate, and potassium persulfate.

In some embodiments, an amount of the initiator in step (4) is 0.3%-0.8%, such as, 0.3%, 0.4%, 0.5%, 0.6%, 0.7% or 0.8%, by weight of the aqueous solution of polyvinyl alcohol.

In some embodiments, a mass of the second aqueous phase in step (5) is 5%-20%, such as, 5%, 8%, 10%, 12%, 15%, 18% or 20%, of mass of the first aqueous phase.

In some embodiments, the oil-soluble dispersant in step (6) is selected from cellulose acetate butyrate.

In some embodiments, the oil phase in step (6) is obtained by adding the oil-soluble dispersant to an oil-based solvent and stirring at 30 °C-50 °C (such as, 30 °C, 33 °C, 35 °C, 38 °C, 40 °C, 45 °C, 48 °C or 50 °C) until dissolved to form a homogeneous solution.

In some embodiments, a mass percentage concentration of the oil-soluble dispersant in the oil phase is 2%-5%, such as, 2%, 2.5%, 3%, 3.5%, 4%, 4.5% or 5%.

In some embodiments, the oil-based solvent in the oil phase is at least one selected from the group consisting of butyl acetate, ethyl acetate, methyl acetate, and propyl acetate, preferably butyl acetate.

In some embodiments, the reverse-phase suspension polymerization in step (6) includes heating the reaction system to 50 °C-80 °C (such as, 60 °C, 63 °C, 65 °C, 68 °C or 70 °C), adding a catalyst to the system, and reacting for 10 h-20 h (such as, 10 h, 13 h, 15 h, 18 h or 20 h).

In some embodiments, the catalyst is selected from tetramethylethylenediamine.

In some embodiments, the reverse-phase suspension polymerization in step (6) is performed under stirring, and a rate of the stirring is 200 rpm-800 rpm, such as, 200 rpm, 230 rpm, 250 rpm, 280 rpm, 300 rpm, 350 rpm, 400 rpm, 450 rpm, 500 rpm, 550 rpm, 600 rpm, 650 rpm, 700 rpm, 750 rpm or 800 rpm.

In some embodiments, a particle size of the core-shell structured polyvinyl alcohol embolic microsphere is 40 µm-1,400 µm. Depending on actual usage requirements, microspheres can be sieved into microspheres with different sizes as required by using sieves with different mesh sizes, such as 40 µm-70 µm, 70 µm -100 µm, 100 µm -150 µm, 150 µm -200 µm, 200 µm - 300 µm, 350 µm -450 µm, 480 µm -580 µm, 650 µm -750 µm, 825 µm -975 µm, 1,025µm -1,175 µm, or 1,225 µm -1,375 µm, etc..

In the second aspect, the present application provides a core-shell structured polyvinyl alcohol embolic microsphere prepared by the preparation method according to the first aspect.

In the third aspect, the present application provides a drug-loaded microsphere, and the drug-loaded microsphere includes the above core-shell structured polyvinyl alcohol embolic microsphere and a loaded drug.

Compared with the prior art, the present application has following beneficial effects:

The core-shell structured polyvinyl alcohol embolic microsphere exhibits an extremely fast drug loading rate and an extremely high drug loading, with a drug loading reaching over 95% in 1 min and a maximum drug loading capacity reaching 150 mg.

### BRIEF DESCRIPTION OF DRAWINGS

FIG. 1 is a microscopic image (video display system, VHX-950F, Keyence (China) Co., Ltd.) of embolic microspheres prepared in Example 1.
FIG. 2 is a test result of effects of addition amount of an aqueous phase 2 on a drug loading ratio of a microsphere.
FIG. 3 is a test result of a maximum doxorubicin loading capacity of different microspheres.
FIG. 4 is a particle size distribution diagram of microspheres in Example 1.
FIG. 5 is a particle size distribution diagram of microspheres of commercially available product 1.
FIG. 6 is a particle size distribution diagram of microspheres of commercially available product 2.
FIG. 7 is a result image of investigation on embolic effect of microspheres in Example 1 in animals.
FIG. 8 is a result of investigation on drug release situation from microspheres in Example 1 in a liver tissue of an embolized site of animals.

### DETAILED DESCRIPTION OF EMBODIMENTS

The technical solution of the present application will be further described below through specific embodiments. Those skilled in the art should understand that the embodiments are merely intended to assist in understanding the present application and should not be regarded as specific limitations on the present application.

### Example 1

In the present example, a core-shell structured polyvinyl alcohol embolic microsphere was prepared, wherein the preparation method included following steps:

### Step 1: Preparation of aqueous solution of low-grafting-degree polyvinyl alcohol

A polymer (polyvinyl alcohol, 100 g) was added to water (500 mL), heated to 95 °C and stirred until dissolved to form a homogeneous solution; then a water-soluble crosslinking agent (N-(2,2-dimethoxy)-2-methacrylamide, 4.00 g) was added, and then an acid catalyst (hydrochloric acid, 30 mL) was added after stirring uniformly. The reaction was carried out under continuous stirring at 25 °C for 10 h, yielding an aqueous solution of polyvinyl alcohol.

### Step 2: Preparation of aqueous solution of high-grafting-degree polyvinyl alcohol

A polymer (polyvinyl alcohol, 100 g) was added to water (500 mL), heated to 95 °C and stirred until dissolved to form a homogeneous solution; then a water-soluble crosslinking agent (N-(2,2-dimethoxy)-2-methacrylamide, 16.00 g) was added, and then an acid catalyst (hydrochloric acid, 30 mL) was added after stirring uniformly. The reaction was carried out under continuous stirring at 25 °C for 10 h, yielding an aqueous solution of polyvinyl alcohol.

### Step 3: Preparation of reverse-phase suspension polymerization oil phase

A dispersant (cellulose acetate butyrate, 20 g) was added to an oil-based solvent (butyl acetate, 1,000 mL), and stirred at 35 °C until dissolved to form a homogeneous solution, yielding an oil phase.

### Step 4: Preparation of system for reverse-phase suspension polymerization water phase 1

A water-soluble monomer with ionic functional groups (sodium 2-acrylamido -2-methylpropanesulfonate, 20 g), an initiator (ammonium persulfate, 1 g), and an aqueous solution of low-grafting-degree polyvinyl alcohol (200 g) were mixed and stirred until a homogeneous solution was obtained, thus yielding an aqueous phase 1.

### Step 5: Preparation of system for reverse-phase suspension polymerization water phase 2

An initiator (ammonium persulfate, 1g), and an aqueous solution of high-grafting-degree polyvinyl alcohol (200 g) were mixed and stirred until a homogeneous solution was obtained, thus yielding an aqueous phase 2.

### Step 6: Preparation of mixed water phase

The aqueous phase 1 (200 g) and the aqueous phase 2 (20 g) were mixed homogeneous to obtain a mixed aqueous phase.

### Step 7: Reverse-phase suspension polymerization

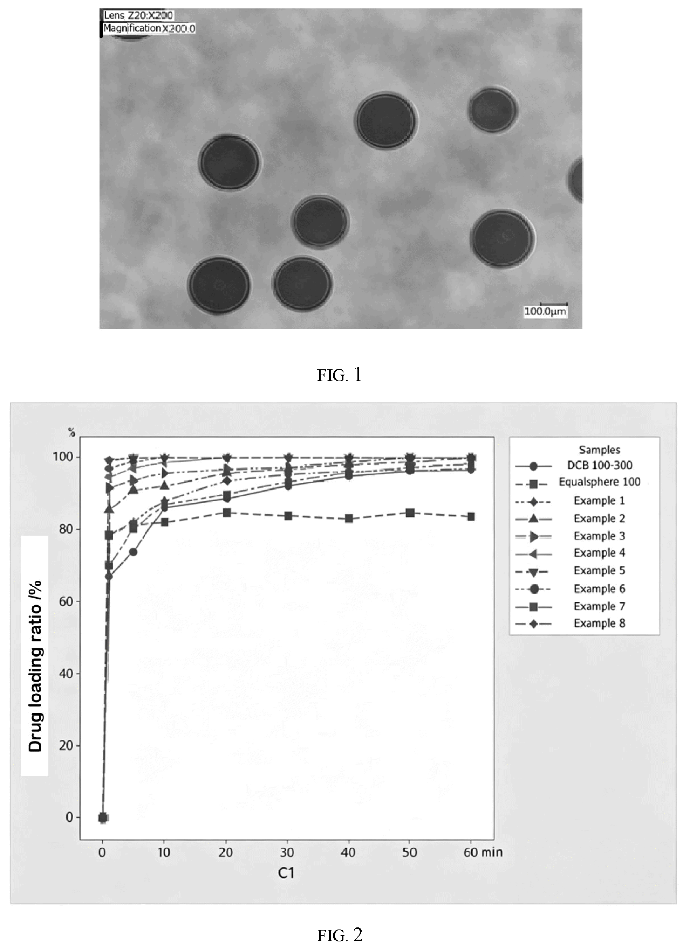

Under a condition of stirring, the mixed aqueous phase solution was slowly added dropwise to the oil phase to form a water-in-oil reverse suspension polymerization system. After the addition was completed, the reaction system was heated to 65 °C, and a catalyst (tetramethylethylenediamine, 5 mL) was added dropwise to the reaction system. The reverse suspension polymerization reaction started and proceeded for 15 h. After the reaction was completed, stirring and heating were stopped, and the reaction system was allowed to stand to separate into layers. The oil phase was separated, and the microsphere was collected and repeatedly washed to obtain embolic microspheres with a core-shell structure (as shown in FIG. 1), with a particle size between 40 µm - 1,400 µm.

### Example 2

The present example provided an embolic microsphere, the preparation method thereof differed from that in Example 1 in that: in step 6, the addition amount of the aqueous phase 2 was 5 g.

### Example 3

The present example provided an embolic microsphere, the preparation method thereof differed from that in Example 1 in that: in step 6, the addition amount of the aqueous phase 2 was 10 g.

### Example 4

The present example provided an embolic microsphere, the preparation method thereof differed from that in Example 1 in that: in step 6, the addition amount of the aqueous phase 2 was 15 g.

### Example 5

The present example provided an embolic microsphere, the preparation method thereof differed from that in Example 1 in that: in step 6, the addition amount of the aqueous phase 2 was 25 g.

### Example 6

The present example provided an embolic microsphere, the preparation method thereof differed from that in Example 1 in that: in step 6, the addition amount of the aqueous phase 2 was 30 g.

### Example 7

The present example provided an embolic microsphere, the preparation method thereof differed from that in Example 1 in that: in step 6, the addition amount of the aqueous phase 2 was 0 g.

### Example 8

The present example provided an embolic microsphere, the preparation method thereof differed from that in Example 1 in that: in step 6, the addition amount of the aqueous phase 2 was 40 g.

### Example 9

The present example provided an embolic microsphere, the preparation method thereof differed from that in Example 1 in that: in step 1, the addition amount of the water-soluble crosslinking agent N-(2,2-dimethoxy)-2-methacrylamide was 2 g; and in step 2, the addition amount of the water-soluble crosslinking agent N-(2,2-dimethoxy)-2-methacrylamide was 20 g.

### Example 10

The present example provided an embolic microsphere, the preparation method thereof differed from that in Example 1 in that: in step 1, the addition amount of the water-soluble crosslinking agent N-(2,2-dimethoxy)-2-methacrylamide was 5 g.

### Example 11

The present example provided an embolic microsphere, the preparation method thereof differed from that in Example 1 in that: in step 1, the addition amount of the water-soluble crosslinking agent N-(2,2-dimethoxy)-2-methacrylamide was 6 g; and in step 2, the addition amount of the water-soluble crosslinking agent N-(2,2-dimethoxy)-2-methacrylamide was 16 g.

### Example 12

The present example provided an embolic microsphere, the preparation method thereof differed from that in Example 1 in that: in step 1, the addition amount of the water-soluble crosslinking agent N-(2,2-dimethoxy)-2-methacrylamide was 8 g; and in step 2, the addition amount of the water-soluble crosslinking agent N-(2,2-dimethoxy)-2-methacrylamide was 32 g.

### Test of drug loading

The drug loading rate of embolic microsphere was tested. A test method was: microspheres (100 µm, 2 mL) were mixed with 4 mL of doxorubicin solution (20 mg/mL). Samples were taken at regular intervals every minute, and a residual amount of doxorubicin in supernatant was determined by high-performance liquid chromatography, and a drug loading of microsphere was obtained through conversion and calculation. Test results are shown in Table 1.

**Table 1 Drug Loading Performance of Drug-Loaded Microspheres**

| Example | Aqueous Phase 1 Addition Amount of Crosslinking Agent/g | Aqueous Phase 2 Addition Amount of Crosslinking Agent/g | Addition Amount of Aqueous Phase 2 /g | Drug Loading Ratio /% | Time Required to achieve 95% Drug Loading /min |
|---|---|---|---|---|---|
| 1 | 4 | 24 | 20 | 99.9 | 1 |
| 2 | 4 | 24 | 5 | 99.9 | 21 |
| 3 | 4 | 24 | 10 | 99.9 | 13 |
| 4 | 4 | 24 | 15 | 99.9 | 6 |
| 5 | 4 | 24 | 25 | 99.9 | 1 |
| 6 | 4 | 24 | 30 | 99.9 | 22 |
| 7 | 4 | 0 | 0 | 98.2 | 40 |
| 8 | 4 | 24 | 40 | 96.6 | 30 |
| 9 | 2 | 20 | 20 | 95.2 | 3 |
| 10 | 5 | 24 | 20 | 96.2 | 2 |
| 11 | 6 | 16 | 20 | 95.4 | 3 |
| 12 | 8 | 32 | 20 | 95.0 | 5 |
| Commercially Available Product 1 | N/A | N/A | N/A | 96.3 | 50 |
| Commercially Available Product 2 | N/A | N/A | N/A | 83.6 | N/A |

| | | | | | |
|---|---|---|---|---|---|
| Note: Commercially available product 1: DC Bead, 100 µm-300 µm, Biocompatibles UK Limited; and Commercially available product 2: Equalsphere, 100 µm, Suzhou Hengrui Jialisheng Biomedical Technology Co., Ltd. | | | | | |

FIG. 2 is a test result of effects of addition amount of an aqueous phase 2 on a drug loading ratio of a microsphere.

Conclusion: 1) With increase of the addition amount of the aqueous phase 2, the drug loading rate increases significantly. When the addition amount is greater than 20 g, approximately 95% of a drug can be loaded in 1 min. However, when the addition amount is greater than 40 g, the drug loading rate begins to decrease. An excessively thick shell layer can affect diffusion of the drug into the core. 2) When the addition amount of the crosslinking agent for the aqueous phase 1 is less than 2 g, due to low degree of crosslinking, the core shrinks rapidly after drug loading, resulting in a slower drug loading rate and a reduced drug loading. When the addition amount of the crosslinking agent for the aqueous phase 1 is greater than 8 g, due to excessive high degree of crosslinking, the core becomes relatively dense, affecting drug diffusion and leading to a slower drug loading rate and a reduced drug loading. 3) When the addition amount of the crosslinking agent for the aqueous phase 2 is less than 16 g, due to low degree of crosslinking, the shell shrinks after drug loading, resulting in a slower drug loading rate and a reduced drug loading. When the addition amount of the crosslinking agent for the aqueous phase 2 is greater than 32 g, the shell becomes relatively dense, affecting drug diffusion, leading to a slower drug loading rate and a reduced drug loading.

### Test of shrinkage ratio of microspheres after drug loading:

A drug loading particle size of the embolic microspheres was tested. A test method was as follows: the embolic microspheres were taken, flattened on a glass slide, and placed under a microscope with a video display system (VHX-950F) for test. The particle sizes of 200 microspheres were measured, and an average value was taken. Test results are shown in Table 2.

**Table 2. Shrinkage Ratio of Microspheres After Drug Loading**

| Example | Average Diameter Before Drug Loading/µm | Average Diameter After Drug Loading/µm | Drug Loading Particle Size Shrinkage Ratio/% | Shell Thickness After Drug Loading/µm |
|---|---|---|---|---|
| 1 | 132.4 | 112.3 | 15.2 | 24 |
| 2 | 130.9 | 94.6 | 27.7 | 8 |
| 3 | 128.5 | 97.5 | 24.1 | 13 |
| 4 | 131.6 | 105.8 | 19.6 | 19 |
| 5 | 120.8 | 105.2 | 12.9 | 30 |
| 6 | 126.1 | 113.1 | 10.3 | 38 |
| 7 | 124.4 | 80.6 | 35.2 | 0 |
| 8 | 122.9 | 116.4 | 5.3 | 47 |
| 9 | 119.4 | 100.5 | 15.8 | 24 |
| 10 | 125.6 | 107.6 | 14.3 | 22 |
| 11 | 127.0 | 101.2 | 20.3 | 15 |
| 12 | 122.2 | 114.3 | 6.5 | 42 |
| Commercially Available Product 1 | 182.6 | 121.2 | 33.6 | 0 |
| Commercially Available Product 2 | 123.5 | 85.5 | 30.8 | 0 |

Conclusion: with increase of the addition amount of the aqueous phase 2, the shell layer of the microsphere becomes significantly thicker, and the particle size shrinkage ratio after drug loading decreases significantly.

### Test of maximum drug loading capacity:

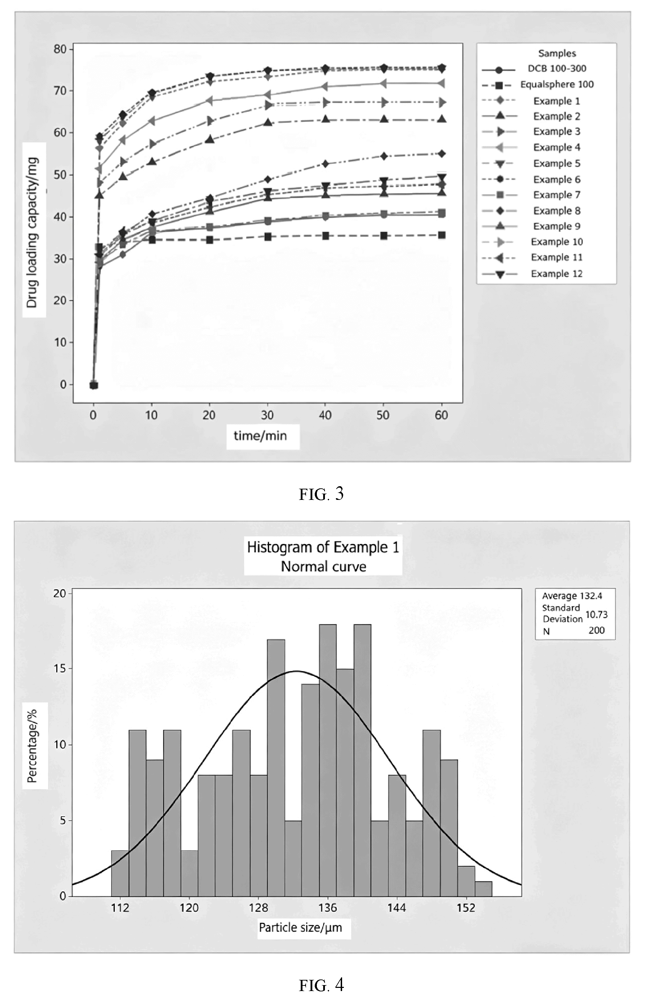

The maximum drug loading capacity of the embolic microsphere was tested and a test method was as follows: microspheres (100 µm, 2 mL) were mixed with 8 mL of doxorubicin solution (20 mg/mL). Samples were taken at regular intervals, and sampling was stopped after the drug solution showed no obvious color change. Residual amount of doxorubicin in supernatant was determined by high-performance liquid chromatography, and a drug loading of the microsphere was obtained through conversion and calculation. Test results are shown in Table 3 (FIG. 3).

**Table 3**

| Example | Aqueous Phase 1 Addition Amount of Crosslinking Agent /g | Aqueous Phase 2 Addition Amount of Crosslinking Agent /g | Addition Amount of Aqueous Phase 2 /g | Maximum Drug Loading Capacity of Microspheres per Milliliter /mg |
|---|---|---|---|---|
| 1 | 4 | 24 | 20 | 75.2 |
| 2 | 4 | 24 | 5 | 63.3 |
| 3 | 4 | 24 | 10 | 67.4 |
| 4 | 4 | 24 | 15 | 71.8 |
| 5 | 4 | 24 | 25 | 75.6 |
| 6 | 4 | 24 | 30 | 75.7 |
| 7 | 4 | 0 | 0 | 41.2 |
| 8 | 4 | 24 | 40 | 55.0 |
| 9 | 2 | 20 | 20 | 65.7 |
| 10 | 5 | 24 | 20 | 64.9 |
| 11 | 6 | 16 | 20 | 59.8 |
| 12 | 8 | 32 | 20 | 58.3 |
| Commercially Available Product 1 | N/A | N/A | N/A | 40.5 |
| Commercially Available Product 2 | N/A | N/A | N/A | 35.5 |

Conclusion: 1) with increase of addition amount of the aqueous phase 2, the shell layer of the microsphere becomes significantly thicker, and shrinkage degree after drug loading becomes small, and a utilization rate of AMPS in the core is high, thus the drug loading is high. 2) When the addition amount of the crosslinking agent for the aqueous phase 1 is 2 g, due to low degree of crosslinking, the core shrinks rapidly after drug loading, resulting in a reduced drug loading. When the addition amount of the crosslinking agent for the aqueous phase 1 is 8 g, due to excessive high degree of crosslinking, the core becomes relatively dense, affecting drug diffusion and leading to a reduced drug loading. 3) When the addition amount of the crosslinking agent for the aqueous phase 2 is 16 g, due to low degree of crosslinking, the shell shrinks after drug loading, resulting in a slower drug loading rate and a reduced drug loading. When the addition amount of the crosslinking agent for the aqueous phase 2 is 32 g, the shell becomes relatively dense, affecting drug diffusion, leading to a slower drug loading rate and a reduced drug loading.

### Test of particle size distribution:

Comparing the particle size distribution of the microspheres prepared in the present application with the particle size distributions of the commercially available products, the embolic microspheres (100 µm -150 µm of particle size range) were taken, flattened on a glass slide, and placed under a microscope using a video display system (VHX-950F) for test. The particle sizes of 200 microspheres were measured, and then a histogram was plotted for comparison.

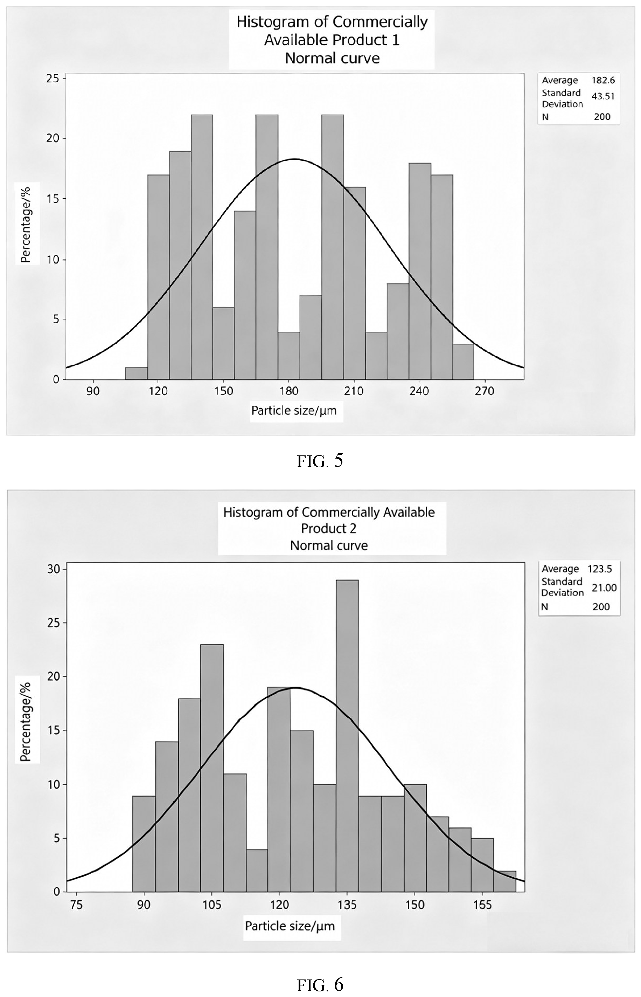

FIG. 4 is a particle size distribution diagram of the microspheres in Example 1, FIG. 5 is a particle size distribution diagram of the microspheres of commercially available product 1, and FIG. 6 is a particle size distribution diagram of the microspheres of commercially available product 2.

It can be seen that a standard deviation of the particle size of 200 microspheres prepared in the present application is smaller than those of the commercially available products, and the particle size distribution is narrower than those of the commercially available products.

The microsphere (100 µm -150 µm) of Example 1 were combined with doxorubicin hydrochloride (25 mg/mL), a common anthracycline chemotherapy drug, to simulate clinical use thereof. A left lobe of a hepatic artery in healthy pigs was embolized via TACE (transcatheter arterial chemoembolization) interventional surgical operation. Effectiveness of the microspheres was evaluated by examining intraoperative operability, immediate and intra-experimental embolization effects, and postoperative blood drug concentration and tissue drug concentrations, etc., of the microspheres. Safety of the microspheres during the experimental period was preliminarily evaluated by observing postoperative recovery condition of the animals and combining with clinical pathology, gross anatomical observation, and histopathological observations, etc. Observation time points were set at 1 day, 7 days, and 28 days after operation, with at least 3 animals at each time point.

All animal surgeries were successfully completed without any adverse phenomena such as catheter blockage, demonstrating that the microspheres have good operability and microcatheter passability. The animals were in good condition after operation, with no accidental deaths.

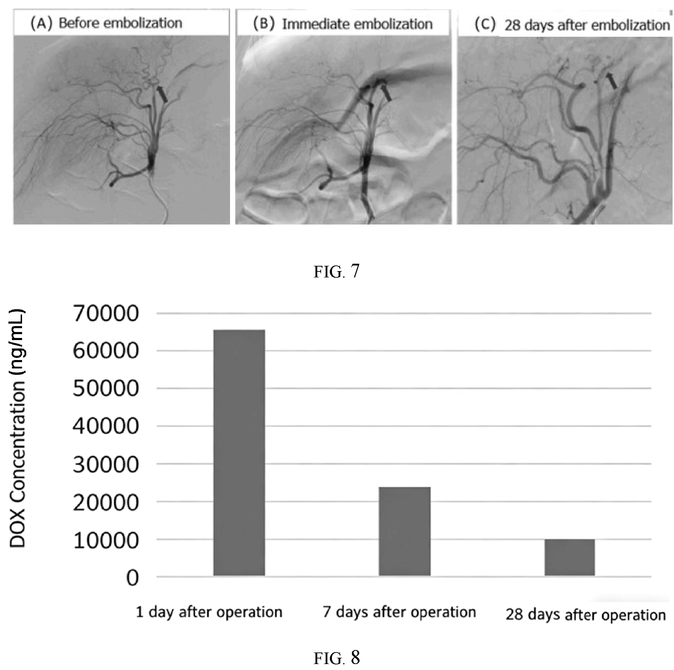

Compared with before embolization, the embolization effect (staining disappears) of a target vessel can be clearly observed in DSA images immediately after embolization, 1 day after operation, 7 days after operation, and 28 days after operation. FIG. 7 shows some data, indicating that the embolization effect is well maintained (arrows indicate embolization sites).

Blood samples were collected from the animals at different time points before and after operation to test hematology, serum biochemistry, blood coagulation, etc. Except for slight fluctuations in a few indicators within 1 week after operation due to effects of drug toxicity and embolization surgery, the rest indicators are normal.

Gross anatomical results showed that, apart from obvious macroscopic lesions at the embolization site (left lateral lobe of the liver), no abnormal phenomena are observed in other lobes of the liver, and other organs (heart, spleen, lungs, kidneys, brain, and gallbladder) are all also normal, indicating that the microsphere has excellent targeting properties and no off-target drift occurs after operation.

Liver tissues from the embolization site of the animals were collected for tissue homogenization, and drug concentrations in the tissues were tested. Tissue concentrations are shown in FIG. 8. It can be seen that the drug concentrations in the tissues gradually decreased over time, remaining at 9,970 ng/mL at 28 days after operation, indicating that the drug is slowly released from the microsphere *in vivo*, maintaining an effective concentration for a relatively long period, demonstrating a good sustained-release effect.

Pathological sections show significant vascular damage at the embolization site, and mesenchymal necrosis or transmural necrosis occurs in a vascular wall, with or without inflammatory cell infiltration, and perivascular tissue necrosis. This is mainly due to the combined effects of the embolization effect and the toxicity of the drug released from the microspheres, proving the effectiveness of the microspheres at the embolization site.

Through the above animal experiments, the good operability of the microspheres is further demonstrated. They exhibit good embolization effect, drug sustained-release effect, and good safety *in vivo*, and are expected to have good efficacy in clinical tumor treatment.

Applicant declares that the present application illustrates the process method through the above examples, but the present application is not limited to the above process steps, that is, it does not mean that the present application must rely on the above process steps to be implemented. Those skilled in the art should understand that any improvements to the present application, equivalent substitutions of raw materials used in the present application, additions of auxiliary components, and selection of specific methods, etc., all fall within the scope of protection and application of the present application.

## Claims

1. A preparation method for core-shell structured polyvinyl alcohol embolic microsphere, comprising following steps:
(1) reacting polyvinyl alcohol with a water-soluble crosslinking agent accounting for 2%-8% by mass of the polyvinyl alcohol to obtain an aqueous solution of low-grafting-degree polyvinyl alcohol;
(2) reacting polyvinyl alcohol with a water-soluble crosslinking agent accounting for 16%-32% by mass of the polyvinyl alcohol to obtain an aqueous solution of high-grafting-degree polyvinyl alcohol;
(3) mixing a water-soluble monomer with ionic functional groups, an initiator and the aqueous solution of low-grafting-degree polyvinyl alcohol obtained in step (1) to obtain a first aqueous phase;
(4) mixing an initiator with the aqueous solution of high-grafting-degree polyvinyl alcohol obtained in step (2) to obtain a second aqueous phase;
(5) mixing the first aqueous phase and the second aqueous phases to obtain a mixed aqueous phase; and
(6) adding the mixed aqueous phase obtained in step (5) to an oil phase containing an oil-soluble dispersant to form a water-in-oil system for reverse-phase suspension polymerization, and performing reverse-phase suspension polymerization to obtain the core-shell structured polyvinyl alcohol embolic microsphere.

2. The preparation method for core-shell structured polyvinyl alcohol embolic microsphere according to claim 1, wherein, before the reaction in step (1), polyvinyl alcohol is added to water, heated to 90 °C-100 °C, and stirred until dissolved to form a homogeneous polyvinyl alcohol solution.

3. The preparation method for core-shell structured polyvinyl alcohol embolic microsphere according to claim 1 or 2, wherein a mass percentage concentration of polyvinyl alcohol in the polyvinyl alcohol solution is 12%-22%.

4. The preparation method for core-shell structured polyvinyl alcohol embolic microsphere according to any one of claims 1 to 3, wherein the water-soluble crosslinking agents in step (1) and step (2) are each independently at least one selected from the group consisting of N-(2,2-dimethoxy)-2-methacrylamide, N-(1-hydroxy-2,2-dimethoxyethyl)-2-acrylamide, N-(2,2-dimethoxy)-2-acrylamide, N-(1-hydroxy-2,2-dimethoxyethyl)-2-methacrylamide, N-(2,2-dimethoxy)-2-methacrylate, N-(1-hydroxy-2,2-dimethoxyethyl)-2-acrylate, N-(2,2-dimethoxy)-2-acrylate, and N-(1-hydroxy-2,2-dimethoxyethyl)-2-methacrylate.

5. The preparation method for core-shell structured polyvinyl alcohol embolic microsphere according to any one of claims 1 to 4, wherein the reaction in step (1) is performed in the presence of an acid catalyst;
preferably, the acid catalyst is at least one of hydrochloric acid, sulfuric acid, nitric acid, or p-toluenesulfonic acid, preferably hydrochloric acid;
preferably, a mass percentage concentration of the acid catalyst in a reaction system is 6%-12%; and
preferably, a temperature of the reaction in step (1) is 20 °C-30 °C, and a duration of the reaction is 10 h-15 h.

6. The preparation method for core-shell structured polyvinyl alcohol embolic microsphere according to any one of claims 1 to 5, wherein, before the reaction in step (2), polyvinyl alcohol is added to water, heated to 90 °C-100 °C, and stirred until dissolved to form a homogeneous polyvinyl alcohol solution;
preferably, a mass percentage concentration of polyvinyl alcohol in the polyvinyl alcohol solution is 12%-22%;
preferably, the reaction in step (2) is performed in the presence of an acid catalyst;
preferably, the acid catalyst is at least one of hydrochloric acid, sulfuric acid, nitric acid, or p-toluenesulfonic acid, preferably hydrochloric acid; and
preferably, a temperature of the reaction in step (2) is 20 °C-30 °C, and a duration of the reaction is 10 h-15 h.

7. The preparation method for core-shell structured polyvinyl alcohol embolic microsphere according to any one of claims 1 to 6, wherein the water-soluble monomer with ionic functional groups in step (3) is at least one selected from the group consisting of sodium 2-acrylamido-2-methylpropanesulfonate, sodium allyl sulfonate, and sodium methallyl sulfonate;
preferably, an amount of the water-soluble monomer with ionic functional groups in step (3) is 5%-20% by weight of the aqueous solution of polyvinyl alcohol;
preferably, the initiator in step (3) is at least one selected from the group consisting of ammonium persulfate, sodium persulfate, and potassium persulfate; and
preferably, an amount of the initiator in step (3) is 0.3%-0.8% by weight of the aqueous solution of polyvinyl alcohol.

8. The preparation method for core-shell structured polyvinyl alcohol embolic microsphere according to any one of claims 1 to 7, wherein the initiator in step (4) is at least one selected from the group consisting of ammonium persulfate, sodium persulfate, and potassium persulfate; and
preferably, an amount of the initiator in step (4) is 0.3%-0.8% by weight of the aqueous solution of polyvinyl alcohol.

9. The preparation method for core-shell structured polyvinyl alcohol embolic microsphere according to any one of claims 1 to 8, wherein a mass of the second aqueous phase is 5%-20% of a mass of the first aqueous phase in step (5);
preferably, the oil-soluble dispersant in step (6) is selected from cellulose acetate butyrate;
preferably, the oil phase in step (6) is obtained by adding the oil-soluble dispersant to an oil-based solvent and stirring at 30 °C-50 °C until dissolved to form a homogeneous solution;
preferably, a mass percentage concentration of the oil-soluble dispersant in the oil phase is 2%-5%;
preferably, the oil-based solvent in the oil phase is at least one selected from the group consisting of butyl acetate, ethyl acetate, methyl acetate, and propyl acetate, preferably butyl acetate;
preferably, the reverse-phase suspension polymerization in step (6) comprises heating a reaction system to 50 °C-80 °C, adding a catalyst to the system, and reacting for 10 h-20 h;
preferably, the catalyst is selected from tetramethylethylenediamine;
preferably, the reverse-phase suspension polymerization in step (6) is performed under stirring, and a rate of the stirring is 200 rpm-800 rpm; and
preferably, a particle size of the core-shell structured polyvinyl alcohol embolic microsphere is 40 µm-1,400 µm.

10. A core-shell structured polyvinyl alcohol embolic microsphere prepared by the preparation method according to any one of claims 1 to 9.

11. A drug-loaded microsphere, comprising the core-shell structured polyvinyl alcohol embolic microsphere according to claim 10 and a loaded drug.
